# EUROPÄISCHE PATENTSCHRIFT

(11) **EP 1 709 990 B1**
(45) Veröffentlichungstag und Bekanntmachung des Hinweises auf die Patenterteilung: **14.01.2009**
(21) Anmeldenummer: 06004689.3
(22) Anmeldetag: 08.03.2006
(51) Int. Cl.: A61N 1/05

(54) **Vorrichtung zum Fixieren einer Elektrode im Schrittmacherbett**
Device for fixing an electrode to the pacemaker pocket
Dispositif pour fixer une électrode dans la poche du stimulateur cardiaque

(30) Priorität: 09.04.2005 DE 102005016364
(43) Veröffentlichungstag der Anmeldung: 11.10.2006
(73) Patentinhaber: Osypka, Peter, Dr.-Ing., 79618 Rheinfelden-Herten (DE)
(72) Erfinder: Osypka, Peter, Dr.-Ing., 79618 Rheinfelden-Herten (DE); Gerstmann, Hans, 79540 Lörrach (DE)
(74) Vertreter: Maucher, Wolfgang

(56) Entgegenhaltungen:
- US-B1- 6 473 654

## Beschreibung

Die Erfindung betrifft eine Vorrichtung zum Fixieren einer Elektrode, insbesondere einer Herzschrittmacherelektrode, im Schrittmacherbett vor dem Eintritt der Elektrode in eine Vene (Ligaturschutz) mit einer Hülse, die eine in axialer Richtung durchgehende Innenhöhlung zur Aufnahme der Elektrode aufweist, welche in ihrem Querschnitt zur Festlegung der Elektrode in Gebrauchsstellung verminderbar ist, wobei die Hülse einen in axialer Richtung gegen eine Rückstellkraft dehnbaren elastischen Schlauch enthält, der gleichzeitig die Innenlängshöhlung zur Aufnahme der Elektrode bildet und die Hülse in Längsrichtung entlang einer an ihrem Umfang verlaufenden Trennstelle in wenigstens zwei in axialer Richtung relativ zueinander bewegbare Teile unterteilt ist.

Eine derartige Vorrichtung ist aus der US-A-5 476 493 bekannt. Dabei sind die beiden Hülsenteile teleskopartig ineinander gesteckt und verschiebbar und zwischen der Stirnseite des innenliegenden Teils und einem Absatz des anderen Teils ist eine Druckfeder eingelegt, deren Kraft die beiden Teile in axialer Richtung unter Dehnung des Schlauches auseinander bewegt, wodurch der Schlauch in seinem mittleren Bereich gleichzeitig aufgrund seiner Dehnung zusammengezogen wird und dadurch die Elektrode klemmend erfasst. Dies stellt eine aufwändige und auch störanfällige Konstruktion dar, die von einer guten Gleitfähigkeit der beiden Teile relativ zueinander und vor allem der zusätzlich darin eingearbeiteten Feder abhängt, deren Montage schwierig ist.

Aus der US-B1-6 473 654 ist eine Vorrichtung zur Fixierung einer Elektrode vergleichbarer Art aber anderer Gattung bekannt, bei welcher zwei relativ zueinander verdrehbare Hülsen in ihrem Inneren eine Schraubenfeder aufweisen, die durch eine gegenseitige Verdrehung in ihrem Querschnitt verändert wird, so dass bei einer Drehrichtung eine vergrößerung und bei der entgegengesetzten Drehrichtung eine Verkleinerung des Querschnitts möglich ist. Somit kann eine Elektrode von dieser Schraubfeder eingeklemmt werden, wobei an der Elektrode durch die Windungen der Feder linienartige Druckstellen auftreten können. Ferner erfordert die genannte Feder eine aufwendige Befestigung mit Hilfe von Bohrungen im Inneren der Hülsen.

Es besteht deshalb die Aufgabe, eine Vorrichtung der eingangs genannten Art zu schaffen, bei welcher eine Druckfeder für eine teleskopartige Verstellung der beiden Teile der Hülse vermieden und dennoch eine gute Verklemmung zwischen dem Schlauch und der Elektrode ermöglicht wird.

Zur Lösung dieser Aufgabe ist die eingangs definierte Vorrichtung dadurch gekennzeichnet, dass die Trennstelle zwischen zwei Teilen der Hülse gegenüber einer Durchmesserebene abwechselnd axial nach entgegengesetzten Seiten abweichend umläuft, so dass die sich stirnseitig berührenden Ränder der Teile der Hülse derart profiliert sind, dass sie in Drehrichtung formschlüssig verbunden sind, und dass die Teile in axialer Richtung entgegen der Rückstellkraft des sie zusammenhaltenden elastischen Schlauches soweit auseinander ziehbar sind, dass die gegenseitige Profilierung gelöst und die beiden Teile unter Tordierung oder Verwringung des elastischen Schlauches verdrehbar und in verdrehter Position wiederum in Berührung mit einander gegen Drehbewegungen festlegbar sind.

Zum Verklemmen der Hülse mit der Elektrode und damit zur axialen Festlegung der Elektrode relativ zu dieser Hülse und damit zu dem Ligaturschutz kann also eine Axialbewegung der beiden Teile durchgeführt werden, wodurch sie soweit voneinander getrennt werden, dass sie relativ zueinander verdreht werden können. Dies führt zu einer Tordierung und damit zu einer Querschnittsverminderung des Schlauches der somit mit seinem Innenquerschnitt die Elektrode kraftschlüssig erfassen kann, wenn die beiden Teile der Hülse nach einer leichten Zugbewegung auseinander bewegt sind und dann relativ zueinander soweit verdreht werden, dass sie nach ihrer erneuten Zusammenführung nun mehr den tordierten Schlauch in ihrem Inneren enthalten. Es leuchtet ein, dass der Durchgang durch einen Schlauch vermindert wird, wenn dieser verdreht oder tordiert wird. Diese Erkenntnis nutzt die Erfindung dazu aus, eine kraftschlüssige Verbindung zwischen der wenigstens zweigeteilten Hülse und einer Elektrode unter Mitwirkung des in der Hülse befindlichen Schlauches zu bewirken.

Dadurch entfällt die Notwendigkeit, dass die beiden Hülsenteile teleskopartig ineinander greifen und in ihrem Inneren eine Druckfeder angeordnet wird. Vielmehr können die Hülsenteile im Bereich der Trennstelle übereinstimmende Querschnitte haben und somit in axialer Richtung bzw. mit den einander zugewandten Stirnseiten direkt oder indirekt aneinander liegen. Durch die Elastizität und die Vorspannung' des Schlauches, die durch die Tordierung bewirkt oder vergrößert wird, werden die beiden Teile ausreichend fest zusammengehalten, so dass die aufgrund der Profilierung aneinander liegenden Stirnseiten dann auch gegen eine Zurückverdrehung gesichert sind.

Zweckmäßig ist es dabei, wenn die Profilierung an der Trennstelle der Hülsenteile aus in axialer Richtung verlaufenden Vertiefungen und Vorsprüngen, Zungen oder dergleichen besteht, wobei die Zungen des einen Teils in die Vertiefungen des anderen Teils insbesondere formschlüssig passen und in Gebrauchsstellung axial eingreifen. Dabei können diese in axialer Richtung verlaufenden Zungen und Vertiefungen derartig geformt sein, dass sie sich ergänzen, so dass die Hülse trotz ihrer Zweiteilung auch im Bereich der Trennstelle eine geschlossene und glatte Oberfläche hat.

Eine Ausführungsform kann vorsehen, dass an dem einen Teil zwei in axialer Richtung vorspringende Zungen vorgesehen sind und dass an dem anderen Teil zwei dazu passende Vertiefungen angeordnet sind, die ihrerseits durch Zungen begrenzt sind, welche zwischen die Zungen des ersten Teils passen. Dabei können die Zungen und Vertiefungen in Umfangsrichtung einen regelmäßigen Abstand zueinander haben und zwei Zungen können um 180°, drei Zungen um 120° usw. gegeneinander versetzt angeordnet sein. Entsprechend große Verdrehbewegungen sind jeweils nach dem axialen Auseinanderziehen der beiden Teile möglich, das heißt lediglich zwei um 180° versetzte Zungen an den beiden Teilen mit dazu passenden Vertiefungen ermöglichen ein Verdrehen der beiden Teile um 180° oder auch um 360° usw. Sind mehr Zungen und Vertiefungen vorhanden, können kleinere Verdrehschritte durchgeführt, dabei aber auch mehrfach durchgeführt werden, um eine ausreichende Tordierung des Schlauches zu bewirken.

Eine abgewandelte Ausführungsform der Erfindung kann darin bestehen, dass die Teile der Hülse an den sich berührenden Rändern eine zueinander passende Verzahnung aufweisen. Einerseits ist dann auch eine relativ geringe Axialbewegung erforderlich, um die beiden Teile aus ihrer gegenseitigen Drehsicherung zu lösen und andererseits sind dann praktisch beliebig kleine oder auch große Relativ-Drehbewegungen möglich, je nachdem, wie groß die Zahnteilung und die Zahnhöhe der gegenseitigen Verzahnung ist.

Dabei können die Zähne der Verzahnung der Teile der Hülse und die Zahnlücken jeweils etwa winkel- oder dreieckförmig ausgebildet sein, so dass die Spitze eines Zahnes in eine entsprechend winkelförmige Zahnlücke passt, woraus sich wiederum eine in Gebrauchsstellung geschlossene Oberfläche der zweigeteilten Hülse ergibt.

An dem elastischen Schlauch können jeweils zu der Hülse gehörende Endanschläge befestigt sein, die als Wiederlager für die zwischen diesen Endanschlägen auf dem Schlauch angeordneten Hülsenteile dienen, wobei zwischen den Endanschlägen und/oder dem Schlauch einerseits und den Teilen der Hülse andererseits jeweils eine Verdrehsicherung vorgesehen ist. Die Endanschläge halten also die Hülsenteile zusammen und die dabei vorgesehene Verdrehsicherung erlaubt die Übertragung der Drehkräfte an den Hülsenteilen über diese Endanschläge auf den Schlauch. Zwischen Endanschlag und Schlauch selbst ist durch deren gegenseitige Befestigung ebenfalls die erforderliche Übertragung der Drehkraft auf den Schlauch sicher gestellt.

An den Endanschlägen kann beispielsweise eine leistenartige Feder in axialer Richtung vorstehen, die in eine stirnseitig offene Nut des zugehörigen Hülsenteils formschlüssig passt oder der Endanschlag kann eine Vertiefung haben, in die ein Vorsprung des Hülsenteils in Gebrauchsstellung eingreift und/oder die Berührstellen der Endanschläge und der Hülsenteile können formschlüssig ineinander passend profiliert sein.

Eine die Tordierung des Schlauches erleichternde vorteilhafte Ausgestaltung der Erfindung kann vorsehen, dass der elastische Schlauch einen oder mehrere in seiner entspannten Lage insbesondere axial über wenigstens einen Teil seiner Länge verlaufende Schlitze aufweist, der/die beim Verdrehen der Hülsenteile etwa zu einer oder mehreren Schraubenlinien verformt wird/werden. Durch eine derartige Schlitzung des Schlauches kann erreicht werden, dass auch nach seiner Tordierung im Inneren flächenartig sich an die Elektrode andrückende Bereiche entstehen und damit die Klemmkraft möglichst gleichmäßig auf dem Umfang der Elektrode verteilt wird.

Günstig ist es, wenn die Außenseite der Hülsenteile aufgeraut oder mit Rillen oder Riefen oder Profilierungen versehen ist, so dass ein Benutzer die beiden Hülsenteile erfassen und auch gegen eine größere Wiederstandskraft gut axial auseinander ziehen und zueinander verdrehen kann, ohne abzurutschen. Es kann aber auch ein Werkstoff gewählt werden, der einen entsprechend hohen Reibungskoeffizienten hat.

Es sei noch erwähnt, dass an der Außenseite wenigstens eines der Hülsenteile, bevorzugt an beiden Hülsenteile eine Ringnut oder dergleichen zum Anbringen von Nahtmaterial zum Fixieren der Vorrichtung im Schrittmacherbett vorgesehen sein kann.

Dabei können derartige Ringnuten ebenfalls das Erfassen und axiale Auseinanderziehen gegen die Rückstellkraft des Schlauches erleichtern.

Vor allem bei Kombination einzelner oder mehrerer der vorbeschriebenen Merkmale und Maßnahmen ergibt sich eine Vorrichtung mit einer zweigeteilten Hülse und einem darin befindlichen Schlauch, die relativ einfach im Aufbau ist, weil eine teleskopartige Verschiebbarkeit der Hülsenteile nicht notwendig ist und keine sie auseinander bewegende Druckfeder im Inneren benötigt wird. Vielmehr kann der Benutzer eine gegenseitige in Drehrichtung wirksame Profilierung und Kupplung durch ein axiales Auseinanderziehen der beiden Teile lösen, diese dann relativ zueinander verdrehen, dadurch den im Inneren befindlichen Schlauch tordieren und seinen Querschnitt vermindern, so dass eine dort verlaufende Elektrode kraftschlüssig erfasst beziehungsweise festgelegt wird.

Nachstehend sind Ausführungsbeispiele der Erfindung anhand der Zeichnung näher beschrieben. Es zeigt in zum Teil schematisierter Darstellung:

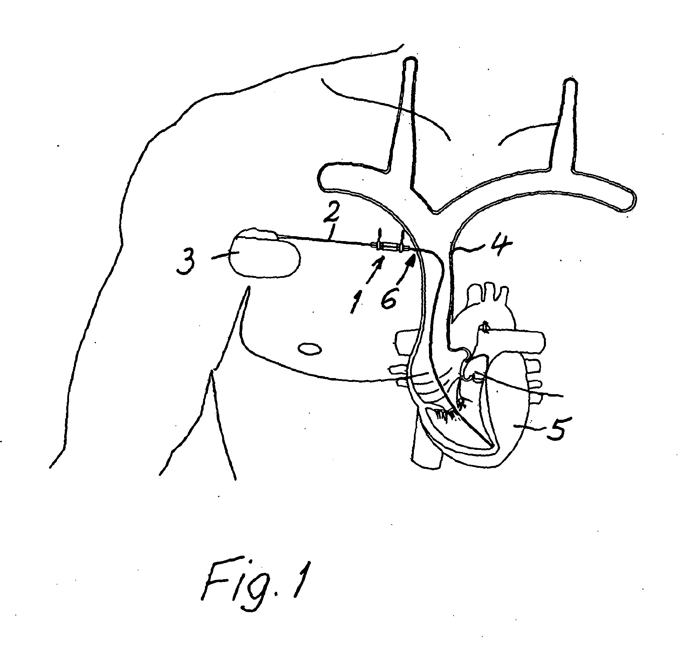

- Fig. 1: einen Herzschrittmacher und eine von diesem durch das Schrittmacherbett zu einer Vene und durch diese in das Innere das Herzens eines Patienten verlaufende Herzschrittmacherelektrode, die kurz vor dem Eintritt in die Vene mit einer erfindungsgemäßen Vorrichtung festgelegt ist,
- Fig. 2: eine Seitenansicht einer die Vorrichtung im Wesentlichen bildenden, in Umfangsrichtung zweigeteilten Hülse, in deren Inneren ein elastisch dehnbarer Schlauch verläuft, der die Innenlängshöhlung zur Aufnahme der Elektrode bildet, wobei die beiden Hülsenteile in axialer Richtung nebeneinander Zungen und Vertiefungen haben, die formschlüssig ineinander passen,
- Fig. 3: eine der Figur 2 entsprechende Darstellung, wobei die beiden Hülsenteile in axialer Richtung soweit auseinander gezogen sind, dass die jeweiligen Zungen aus den Vertiefungen heraus bewegt sind, so dass die Hülsenteile relativ zueinander verdreht werden können, bevor sie dann wieder axial zusammenpassend zusammen geführt werden, wobei durch die Verdrehung der im Inneren befindliche elastische Schlauch tordiert und in seinem Querschnitt vermindert wird, so dass er eine Elektrode kraftschlüssig erfassen kann,
- Fig. 4: den elastischen Schlauch mit Endanschlägen ohne die beiden Hülsenteile,
- Fig. 5: einen Hülsenteil unabhängig von dem Schlauch und den Endanschlägen,
- Fig. 6: die Anordnung des einen Hülsenteils auf dem Schlauch und in Relation zu dem Endanschlag und
- Fig. 7: schließlich die fertige Hülse mit den beiden an ihren Berührstellen formschlüssig ineinander greifenden Hülsenteilen, die an den entgegengesetzten Enden von den Endanschlägen des Schlauches gehalten sind,
- Fig. 8: eine Ansicht einer abgewandelten Ausführungsform, bei welcher die beiden Hülsenteile an ihrer Berührstelle eine Verzahnung miteinander haben,
- Fig. 9: eine der Figur 8 entsprechende Darstellung nach dem zunächst axialen Auseinanderziehen, Verdrehen und dann wieder Zusammenführen der beiden Hülsenteile, wodurch der im Inneren befindliche Schlauch tordiert und sein Innenquerschnitt vermindert wurde,
- Fig. 10: einen Hülsenteil der Vorrichtung gemäß Figur 8 und 9,
- Fig. 11: den im Inneren der Hülse befindlichen Schlauch mit einem Endanschlag und einem der beiden Hülsenteile, das bereits in Gebrauchsstellung ist, sowie
- Fig. 12: in schaubildlicher Darstellung die zweigeteilte Hülse, deren beiden Teile an ihrer Berührstelle in Drehrichtung miteinander verzahnt sind.

Eine im Ganzen mit 1 bezeichnete Vorrichtung dient zum Fixieren einer Elektrode 2, im Ausführungsbeispiel einer Herzschrittmacherelektrode, im Schrittmacherbett, wobei die Elektrode 2 von einem Herzschrittmacher 3 durch das Schrittmacherbett zu einer Vene 4 und durch diese hindurch in das Herz 5 verläuft und sich die Vorrichtung 1 kurz vor dem Eintritt 6 in die Vene 4 befindet. Diese als Ligaturschutz dienende Vorrichtung 1 weist dabei eine aus zwei Hülsenteilen 7 und 8 bestehende Hülse 9 auf, die eine in axialer Richtung durchgehende Innenhöhlung zur Aufnahme der Elektrode 2 hat, wobei diese Innenhöhlung in ihrem Querschnitt zur Festlegung der Elektrode 2 in Gebrauchsstellung verminderbar ist, so dass dann Kraftschluss zwischen der Elektrode 2 und der Innenhöhlung bzw. den Hülsenteilen 7 und 8 besteht.

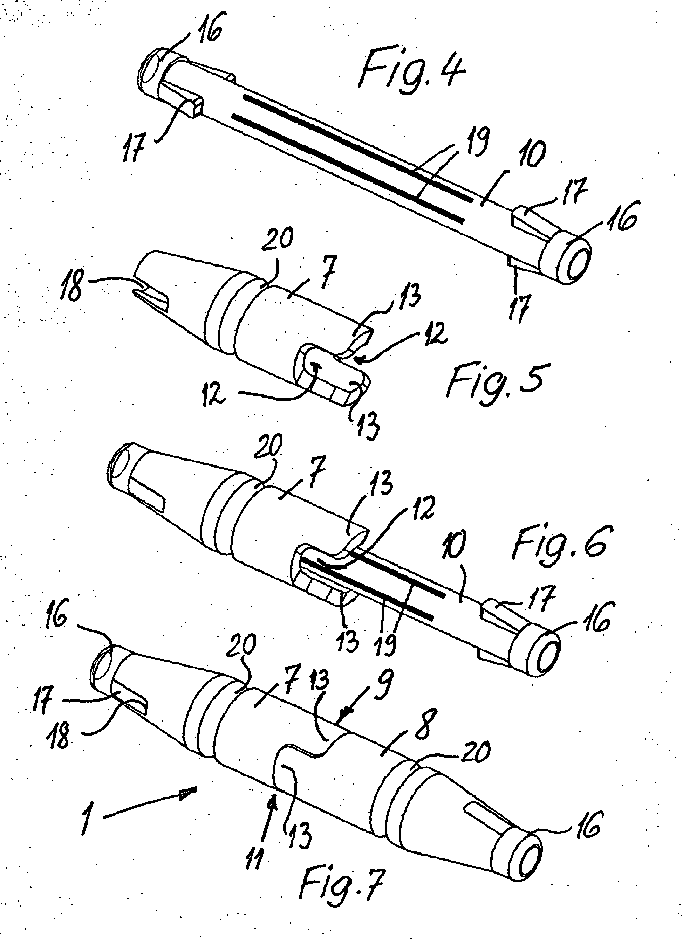

Dabei enthält die im Ganzen mit 9 bezeichnete Hülse einen in axialer Richtung gegen eine Rückstellkraft bzw. die Elastizität seines Materials dehnbaren elastischen Schlauch 10, der besonders gut in den Figuren 4, 6 und 11 erkennbar ist und der gleichzeitig die Innenlängshöhlung zur Aufnahme der Elektrode 2 bildet.

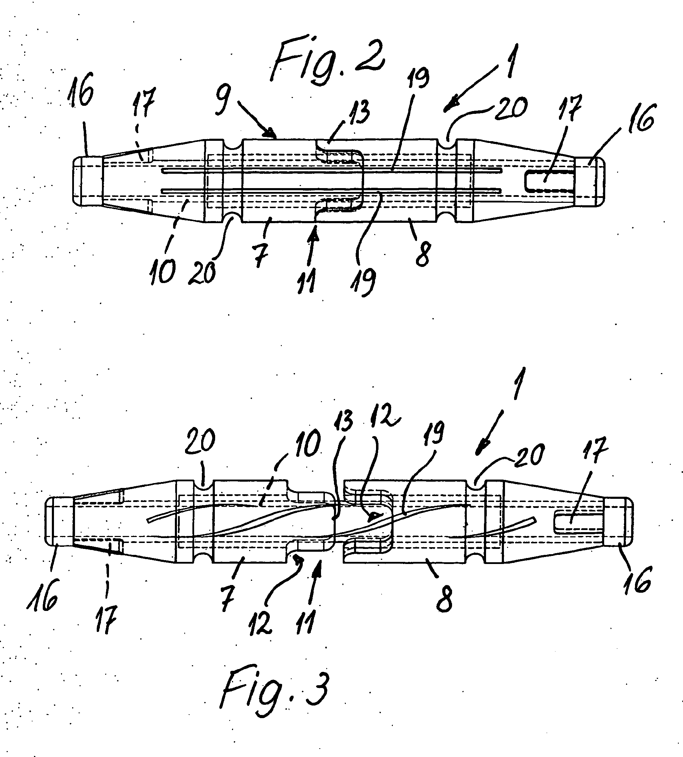

Die Hülse 9 ist in Längsrichtung entlang einer an ihrem Umfang umlaufenden Trennstelle 11 in die beiden in axialer Richtung relativ zueinander bewegbaren Teile 7 und 8 unterteilt, das heißt, gemäß Figur 3 können die beiden Hülsenteile 7 und 8 entgegen der Rückstellkraft des Schlauches 10 auseinander gezogen werden, so dass sie sich an ihrer Trennstelle 11 nicht mehr berühren.

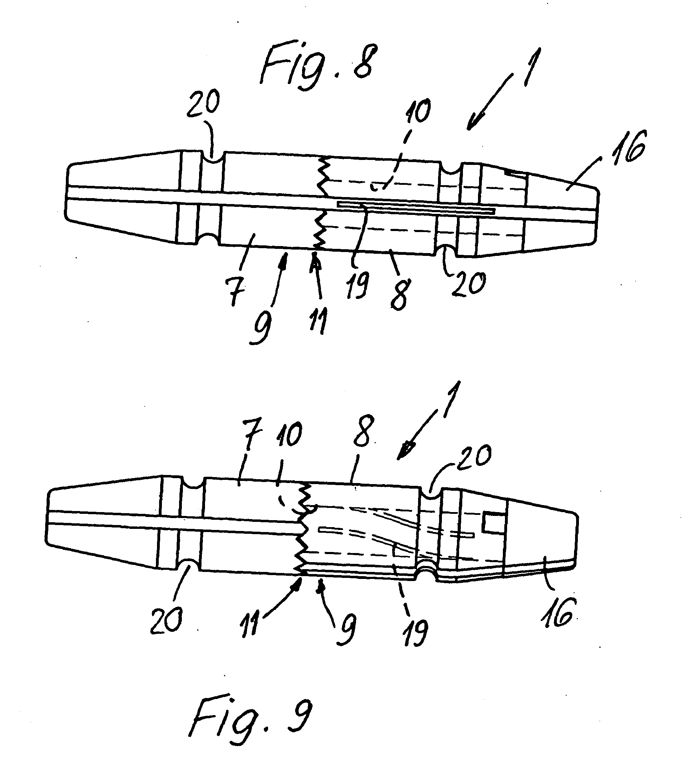

Dabei ist in beiden Ausführungsbeispielen vorgesehen, dass die Trennstelle 11 gegenüber einer Durchmesserebene abwechselnd axial nach entgegengesetzten Seiten abweichend umläuft, so dass die sich stirnseitig berührenden Ränder der Teile 7 und 8 der Hülse 9 derart profiliert sind, dass sie in Drehrichtung formschlüssig verbunden sind, und dass die Teile 7 und 8 durch die schon erwähnte Auseinanderbewegung in axialer Richtung aus ihrer gegenseitigen in Drehrichtung wirkenden Kupplung gelöst werden können und zwar soweit, dass die beiden Teile nunmehr unter Tordierung oder Verwringung des elastischen Schlauches 10 verdreht und dann in verdrehter Position wieder in die Kupplungsstellung und in gegenseitige Berührung miteinander gebracht werden können, in der sie wiederum gegen Drehbewegungen festgelegt sind. Man erkennt beim Vergleich der Figuren 2 und 3 deutlich, wie die beiden Teile 7 und 8 auseinander gezogen und dann verdreht werden können, wobei Figur 3 eine Position zeigt, bei welcher die gegenseitigen Profilierungen gerade wieder zusammen passen aber noch nicht zusammengeführt sind. Analoges gilt für die Figuren 8 und 9 bei einem abgewandelten Ausführungsbeispiel.

Im Ausführungsbeispiel gemäß Figur 2 bis 7 besteht die Profilierung an der Trennstelle 11 der Hülsenteile 7 und 8 aus in axialer Richtung verlaufenden Vertiefungen 12 und Vorsprüngen oder Zungen 13, wobei die Zungen 13 des einen Teils 7 in die Vertiefungen 12 des anderen Teils 8 und ausserdem die Zungen 13 des Teils 8 in die Vertiefungen 12 des Teils 7 formschlüssig passen und zwar konturenmäßig so, dass sich gemäß Figur 7 trotz dieser Trennstelle 11 eine praktisch glatte und durchgehende oder geschlossene Oberfläche der Hülse 9 ergibt.

Dabei sind an dem einen Teil 7 zwei in axialer Richtung vorspringende und in Umfangsrichtung etwas gekrümmte Zungen 13 und an dem anderen Teil 8 zwei dazu passende Vertiefungen 12 angeordnet, die ihrerseits durch Zungen 13 begrenzt sind, welche zwischen die Zungen 13 des ersten Teils 7 passen. Vor allem anhand der Figuren 5 bis 7 wird deutlich, dass praktisch die Zungen 13 die selben Konturen wie die Vertiefungen 12 haben, so dass sie sich jeweils entsprechend ergänzen können. Dabei erkennt man ferner, dass diese Zungen 13 und die Vertiefungen 12 in Umfangsrichtung einen regelmäßigen Abstand zueinander haben und die Zungen 13 und damit auch die Vertiefungen 12 jeweils um 180° gegeneinander versetzt sind. Es könnten aber auch mehr Zungen und Vertiefungen mit einem geringeren Winkelabstand zueinander vorgesehen sein.

Im Ausführungsbeispiel gemäß den Figuren 8 bis 12 weisen die Teile 7 und 8 der Hülse 9 an den sich berührenden Rändern eine zueinander passende Verzahnung als gegenseitige Profilierung auf. Man kann deutlich erkennen, dass diese relativ zahlreichen Zähne 14 eine nur geringe axiale Bewegung der beiden Teile 7 und 8 voneinander weg notwendig machen und dass die gegenseitige Verdrehung in kleinen oder auch größeren Schritten erfolgen kann, um den im Inneren befindlichen Schlauch 10 zu tordieren und dadurch kraftschlüssig an die Elektrode 2 anzudrücken.

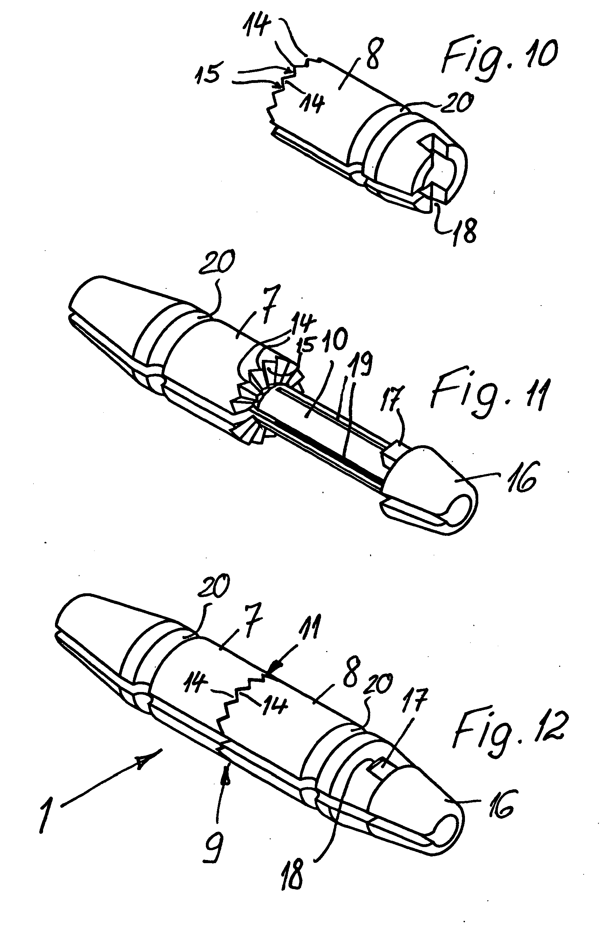

Die Zähne 14 der Verzahnung der Teile 7 und 8 der Hülse 9 und die Zahnlücken 15 (Vergleiche insbesondere Figur 10) sind im Ausführungsbeispiel jeweils etwa winkel- oder dreieckförmig ausgebildet, so dass die Spitze eines Zahnes 14 in eine entsprechend winkelförmige Zahnlücke 15 passt und wiederum in Gebrauchsstellung eine glatte und geschlossene Oberfläche der Hülse 9 ergibt.

Bei beiden Ausführungsbeispielen ist vorgesehen, dass an dem elastischen Schlauch 10 jeweils zu der Hülse 9 bzw. zu den Hülsenteilen 7 und 8 gehörende Endanschläge 16 befestigt sind, die als Wiederlager für die zwischen diesen Endanschlägen 16 auf dem Schlauch 10 befindlichen Hülsenteile 7 und 8 dienen, wobei zwischen den Endanschlägen 16 und/oder dem Schlauch 10 einerseits und den Teilen 7 und 8 der Hülse 9 andererseits jeweils eine Verdrehsicherung vorgesehen ist, so dass das Auseinanderziehen der Hülsenteile 7 und 8 in axialer Richtung über die Endanschläge 16 zu einer axialen Dehnung des Schlauches 10 führt, während anschließend die Relativverdrehung der Teile 7 und 8 zueinander wiederum über die Endanschläge 16 auf den Schlauch 10 übertragen werden kann, so dass dieser in der gewünschten Weise tordiert und dadurch sein Innenquerschnitt zum klemmenden oder kraftschlüssigen Erfassen der Elektrode 2 vermindert wird.

Man erkennt an den Endanschlägen 16 jeweils eine leistenartige Feder 17, die in axialer Richtung vorsteht und dabei gleichzeitig in radialer Richtung gegenüber dem Schlauch 10 übersteht und die in eine stirnseitig offene Nut 18 des zugehörigen Hülsenteils 7 oder 8 formschlüssig passt. Denkbar wäre aber auch, dass der Endanschlag 16 eine Vertiefung und das jeweilige Hülsenteil 7 oder 8 einen dazu passenden Vorsprung hat. Schließlich könnten auch die Berührstellen der Endanschläge 16 und der Hülsenteile 7 oder 8 formschlüssig ineinander passend profiliert oder verzahnt sein. Durch diese Maßnahmen ist eine gute Kraftübertragung vor allem beim Verdrehen der Hülsenteile relativ zueinander und dem damit einhergehenden Tordieren des Schlauches 10 möglich.

In den Figuren 2 bis 4, 6, 8, 9 und 11 erkennt man, dass der elastische Schlauch 10 mehrere in seiner entspannten Lage im Ausführungsbeispiel axial über wenigstens einen Teil seiner Länge verlaufende Schlitze 19 aufweist, die beim Verdrehen der Hülsenteile 7 und 8 relativ zueinander aufgrund der dadurch bewirkten Tordierung des Schlauches zu einer oder mehreren Schraubenlinien verformt werden. Diese Art der Verformung zeigen besonders gut einen Vergleich der Figuren 2 und 3 oder der Figuren 8 und 9 miteinander, woraus sich gleichzeitig ergibt, dass Figur 3 eine Verdrehung der beiden Hülsenteile 7 und 8 relativ zueinander und nicht nur deren axiale Bewegung voneinander weg veranschaulicht. An der Außenseite der Hülsenteile 7 und 8 erkennt man jeweils eine Ringnut 20, die einerseits zum Anbringen von Nahtmaterial zum Fixieren der Vorrichtung 1 im Schrittmacherbett dient oder geeignet ist und die gleichzeitig außerdem das Erfassen und axiale Auseinanderziehen der beiden Hülsenteile 7 und 8 begünstigt. Zusätzlich könnte die Außenseite der Hülsenteile 7 und 8 aufgeraut oder mit Rillen oder Riefen oder Profilierungen versehen sein, um die gegenseitige Bewegung der Hülsenteile 7 und 8 entgegen der Rückstellkraft des elastischen Schlauches 10 zu begünstigen.

Ein Operateur kann mit der Vorrichtung 1 also eine Elektrode 2 kraftschlüssig erfassen, wenn diese im Herzen und im Schrittmacherbett bestmöglich positioniert ist und kann sie dann mit Hilfe der Vorrichtung 1 kurz vor dem Eintritt 6 in die Vene 4 in üblicher Weise fixieren.

Die Vorrichtung 1 dient zum Fixieren einer Elektrode 2 im Schrittmacherbett vor dem Eintritt 6 der Elektrode 2 in eine Vene 4 und weist eine aus zwei Teilen 7 und 8 bestehende Hülse 9 auf, in deren Inneren ein Schlauch 10 verläuft. Die beiden Teile 7 und 8 der Hülse 9 sind an den sich berührenden Rändern einer gemeinsamen Trennstelle 10 profiliert, so dass sie in Drehrichtung relativ zueinander so lange festgelegt sind, bis ein Benutzer sie über die axiale Erstreckung der Profilierung hinaus auseinander zieht. Dieser auseinander gezogenen Position können die Hülsenteile unter Tordierung des Schlauches relativ zueinander verdreht und dann aufgrund der Rückstellkraft des Schlauches 10 wieder axial zusammengeführt werden, wodurch der Innenquerschnitt des Schlauches vermindert und eine dort verlaufende Elektrode 2 formschlüssig erfasst ist.

## Patentansprüche

1. Vorrichtung (1) zum Fixieren einer Elektrode (2), insbesondere einer Herzschrittmacherelektrode, im Schrittmacherbett vor dem Eintritt (6) der Elektrode (2) in eine Vene (4) (Ligaturschutz) mit einer Hülse (9), die eine in axialer Richtung durchgehende Innenhöhlung zur Aufnahme der Elektrode (2) aufweist, welche in ihrem Querschnitt zur Festlegung der Elektrode (2) in Gebrauchsstellung verminderbar ist, wobei die Hülse (9) einen in axialer Richtung gegen eine Rückstellkraft dehnbaren elastischen Schlauch (10) enthält, der gleichzeitig die Innenlängshöhlung zur Aufnahme der Elektrode (2) bildet und die Hülse (9) in Längsrichtung entlang einer an ihrem Umfang umlaufenden Trennstelle (11) in wenigstens zwei in axialer Richtung relativ zueinander bewegbare Teile (7, 8) unterteilt ist, **dadurch gekennzeichnet, dass** die Trennstelle (11) gegenüber einer Durchmesserebene abwechselnd axial nach entgegengesetzten Seiten abweichend umläuft, so dass die sich stirnseitig berührenden Ränder der Teile (7, 8) der Hülse (9) derart profiliert sind, dass sie in Drehrichtung formschlüssig verbunden sind, und dass die Teile (7, 8) in axialer Richtung entgegen der Rückstellkraft des sie zusammenhaltenden elastischen Schlauches soweit auseinander ziehbar sind, dass die gegenseitige Profilierung gelöst und die beiden Teile unter Tordierung oder Verwringung des elastischen Schlauches (10) verdrehbar und in verdrehter Position wiederum in Berührung miteinander gegen Drehbewegungen festlegbar sind.

2. Vorrichtung nach Anspruch 1, **dadurch gekennzeichnet, dass** die Profilierung an der Trennstelle (11) der Hülsenteile (7, 8) aus in axialer Richtung verlaufenden Vertiefungen (12) und Vorsprüngen, Zungen (13) oder dergleichen besteht, wobei die Zungen (13) des einen Teils (7) in die Vertiefungen (12) des anderen Teils (8) insbesondere formschlüssig passen.

3. Vorrichtung nach Anspruch 1 oder 2, **dadurch gekennzeichnet, dass** an dem einen Teil (7) zwei in axialer Richtung vorspringende Zungen (13) vorgesehen sind und dass an dem anderen Teil (8) zwei dazu passende Vertiefungen (12) angeordnet sind, die ihrerseits durch Zungen (13) begrenzt sind, welche zwischen die Zungen (13) des ersten Teils (7) passen.

4. Vorrichtung nach einem der Ansprüche 1 bis 3, **dadurch gekennzeichnet, dass** die Zungen (13) und Vertiefungen (12) in Umfangsrichtung einen regelmäßigen Abstand zueinander haben und zwei Zungen (13) um 180°, drei Zungen um 120° und so weiter gegeneinander versetzt angeordnet sind.

5. Vorrichtung nach einem der Ansprüche 1 bis 4, **dadurch gekennzeichnet, dass** die Teile (7, 8) der Hülsen (9) an den sich berührenden Rändern eine zueinander passende Verzahnung aufweisen.

6. Vorrichtung nach einem der Ansprüche 1 bis 5, **dadurch gekennzeichnet, dass** die Zähne (14) der Verzahnung der Teile (7, 8) der Hülse (9) und die Zahnlücken (15) jeweils etwa winkel- oder dreieckförmig ausgebildet sind, so dass die Spitze eines Zahnes (14) in eine entsprechend winkelförmige Zahnlücke (15) passt.

7. Vorrichtung nach einem der Ansprüche 1 bis 6, **dadurch gekennzeichnet, dass** an dem elastischen Schlauch (10) jeweils zu der Hülse (9) gehörende Endanschläge (16) befestigt sind, die als Widerlager für die zwischen diesen Endanschlägen (16) auf dem Schlauch (10) angeordneten Hülsenteile (7, 8) dienen, wobei zwischen den Endanschlägen (16) und/oder dem Schlauch (10), einerseits und den Teilen (7, 8) der Hülse (9) andererseits jeweils eine Verdrehsicherung vorgesehen ist.

8. Vorrichtung nach einem der Ansprüche 1 bis 7, **dadurch gekennzeichnet, dass** an den Endanschlägen (16) eine leistenartige Feder (17) in axialer Richtung vorsteht, die in eine stirnseitig offene Nut (18) des zugehörigen Hülsenteils (7, 8) formschlüssig passt oder dass der Endanschlag (16) eine Vertiefung hat, in die ein Vorsprung des Hülsenteils (7, 8) in Gebrauchsstellung eingreift und/oder dass die Berührstellen der-Endanschläge (16) und der Hülsenteile (7, 8) formschlüssig ineinanderpassend profiliert sind.

9. Vorrichtung nach einem der Ansprüche 1 bis 9, **dadurch gekennzeichnet, dass** der elastische Schlauch (10) einen oder mehrerer in seiner entspannten Lage insbesondere axial über wenigstens einen Teil seiner Länge verlaufende Schlitze (19) aufweist, der/die beim Verdrehen der Hülsenteile (7, 8) etwa zu einer oder mehreren Schraubenlinien verformt wird/werden.

10. Vorrichtung nach einem der Ansprüche 1 bis 9, **dadurch gekennzeichnet, dass** die Außenseite der Hülsenteile (7, 8) aufgeraut oder mit Rillen oder Riefen oder Profilierungen versehen ist.

11. Vorrichtung nach einem der Ansprüche 1 bis 10, **dadurch gekennzeichnet, dass** an der Außenseite wenigstens eines der Hülsenteile (7, 8) eine Ringnut (20) oder dergleichen zum Anbringen von Nahtmaterial zum Fixieren der Vorrichtung (1) im Schrittmacherbett vorgesehen ist.

## Claims

1. Apparatus (1) for fixing an electrode (2), particularly a cardiac pacemaker electrode, in the pacemaker bed before the entry (6) of the electrode (2) into a vein (4) (ligature protection) having a sleeve (9) which comprises an axially extending inner cavity for accommodating the electrode (2), which is reducable in its cross-section in order to secure the electrode (2) in the position of use, the sleeve (9) containing an elastic tube (10) which can be expanded in the axial direction counter to a restoring force and which simultaneously forms the inner longitudinal cavity for accommodating the electrode (2) and the sleeve (9) is sub-divided in the longitudinal direction along a separating point (11) extending around its circumference into at least two parts (7, 8) which are movable relative to one another in the axial direction, **characterised in that** the separating point (11) deviates axially on alternate opposite sides relative to a diametral plane as it runs round, so that the edges of the parts (7, 8) of the sleeve (9) in contact with one another at their end faces are profiled so as to be interlockingly connected to one another in the direction of rotation, and the parts (7, 8) can be pulled apart in the axial direction counter to the restoring force of the elastic tubes that holds them together, to an extent such that the profiling on both sides is released and the two parts can be rotated by twisting or wringing the elastic tube (10) and resecured in the new rotational position in contact with one another against rotary movements.

2. Apparatus according to claim 1, **characterised in that** the profiling at the separating point (11) of the sleeve portions (7, 8) consists of depressions (12) extending in the axial direction and projections, tongues (13) or the like, the tongues (13) of one part (7) fitting, more particularly by interlocking engagement, in the depressions (12) in the other part (8).

3. Apparatus according to claim 1 or 2, **characterised in that** two axially protruding tongues (13) are provided on one part (7) and two mating depressions (12) are arranged on the other part (8), these mating depressions (12) in turn being limited by tongues (13) which fit between the tongues (13) of the first part (7).

4. Apparatus according to one of claims 1 to 3, **characterised in that** the tongues (13) and depressions (12) are regularly spaced from one another in the circumferential direction and two tongues (13) are arranged at 180°, three tongues at 120° and so on, relative to one another.

5. Apparatus according to one of claims 1 to 4, **characterised in that** the parts (7, 8) of the sleeve (9) comprise mating teeth on the edges that come into contact.

6. Apparatus according to one of claims 1 to 5, **characterised in that** the teeth (14) of the set of teeth on the parts (7, 8) of the sleeve (9) and the gaps (15) between the teeth are angular or triangular in shape, so that the point of a tooth (14) fits into a correspondingly angular gap (15).

7. Apparatus according to one of claims 1 to 6, **characterised in that** end fittings (16) belonging to the sleeve (9) are fixed to the elastic tube (10), acting as an abutment for the sleeve parts (7, 8) arranged between these end attachments (16) on the tube (10), while between the end attachments (16) and/or the tube (10) on the one hand and the parts (7, 8) of the sleeve (9) on the other hand there is provided a rotation preventer.

8. Apparatus according to one of claims 1 to 7, **characterised in that** protruding axially from the end attachments (16) is a strip-shaped spring (17) which fits by interlocking engagement in a groove (18) open on the end face of the associated sleeve part (7, 8), or **in that** the end attachment (16) has a depression in which a projection of the sleeve part (7, 8) engages in the position of use and/or **in that** the contacting points of the end attachments (16) and sleeve portions (7, 8) are profiled so as to fit by interlocking engagement into one another.

9. Apparatus according to one of claims 1 to 9, **characterised in that** the elastic tube (10) comprises one or more slots (19) extending, in the relaxed position, in particular axially over at least part of its length, which is/are deformed as the sleeve portions (7, 8) are rotated to form one or more helical lines.

10. Apparatus according to one of claims 1 to 9, **characterised in that** the outside of the sleeve portions (7, 8) is roughened or provided with grooves or channels or profiles.

11. Apparatus according to one of claims 1 to 10, **characterised in that** on the outside of at least one of the sleeve portions (7, 8) is provided an annular groove (20) or the like for attaching suture material for fixing the apparatus (1) in the pacemaker bed.

## Revendications

1. Dispositif (1) pour fixer une électrode (2), en particulier une électrode de stimulateur cardiaque, dans la poche de stimulateur avant l'entrée (6) de l'électrode (2) dans une veine (4) (protection par ligature) avec un manchon (9) qui présente une cavité interne continue en direction axiale pour recevoir l'électrode (2), laquelle est réductible dans sa section pour immobiliser l'électrode (2) en position d'utilisation, le manchon (9) contenant un tuyau élastique (10) extensible en direction axiale à l'encontre d'une force de rappel, qui forme en même temps la cavité interne longitudinale destinée à recevoir l'électrode (2) et le manchon (9) étant divisé en direction longitudinale le long d'une zone de séparation (11) s'étendant tout autour de sa circonférence en au moins deux parties (7, 8) mobiles l'une par rapport à l'autre en direction axiale, **caractérisé en ce que** la zone de séparation (11) fait le tour de la circonférence en s'écartant alternativement vers des côtés opposés axialement par rapport à un plan diamétral, de sorte que les bords frontalement en contact des parties (7, 8) du manchon (9) sont profilés de manière à être en liaison par complémentarité de formes dans le sens de rotation, et que les parties (7, 8) peuvent être séparées en direction axiale à l'encontre de la force de rappel du tuyau élastique qui les maintient ensemble au point que le profilage mutuel peut être détaché et les deux parties être tournées par torsion ou déformation du tuyau élastique (10) et, en position tournée, de nouveau être immobilisées contre les mouvements de rotation en contact l'une avec l'autre.

2. Dispositif selon la revendication 1, **caractérisé en ce que** le profilage à la zone de séparation (11) des parties de manchon (7, 8) est composé de renfoncements (12) et de saillies, languettes (13) ou analogues s'étendant en direction axiale, les languettes (13) d'une partie (7) s'adaptant, en particulier par complémentarité de formes, dans les renfoncements (12) de l'autre partie (8).

3. Dispositif selon la revendication 1 ou 2, **caractérisé en ce que** sur une partie (7) sont prévues deux languettes (13) en saillie en direction axiale et que sur l'autre partie (8) sont disposés deux renfoncements (12) adaptés à celles-ci qui eux-mêmes sont limités par des languettes (13) qui s'adaptent entre les languettes (13) de la première partie (7).

4. Dispositif selon l'une des revendications 1 à 3, **caractérisé en ce que** les languettes (13) et les renfoncements (12) ont une distance mutuelle régulière en direction circonférentielle et deux languettes (13) sont décalées de 180°, trois languettes de 120° et ainsi de suite les unes par rapport aux autres.

5. Dispositif selon l'une des revendications 1 à 4, **caractérisé en ce que** les parties (7, 8) du manchon (9) présentent une denture correspondante l'une à l'autre aux bords qui sont en contact.

6. Dispositif selon l'une des revendications 1 à 5, **caractérisé en ce que** les dents (14) de la denture des parties (7, 8) du manchon (9) et les espaces entre dents (15) présentent chaque fois une forme approximativement angulaire ou triangulaire, de manière que la pointe d'une dent (14) s'adapte dans un espace entre dents (15) de forme angulaire correspondante.

7. Dispositif selon l'une des revendications 1 à 6, **caractérisé en ce qu'**au tuyau élastique (10) sont fixées des butées d'extrémité (16) faisant partie du manchon (9), qui servent de contre-appui pour les parties de manchon (7, 8) disposées entre ces butées d'extrémité (16) sur le tuyau (10), un dispositif de blocage en rotation étant chaque fois prévu entre les butées d'extrémité (16) et/ou le tuyau (10) d'une part et les pièces (7, 8) du manchon (9) d'autre part.

8. Dispositif selon l'une des revendications 1 à 7, **caractérisé en ce que** sur les butées d'extrémité (16) fait saillie en direction axiale un ressort (17) en forme de bande qui s'adapte par complémentarité de formes dans une rainure (18) ouverte frontalement de la partie de manchon (7, 8) associée ou que la butée d'extrémité (16) possède un renfoncement dans lequel une saillie de la partie de manchon (7, 8) s'engage en position d'utilisation et/ou que les zones de contact des butées d'extrémité (16) et des parties de manchon (7, 8) sont profilées de manière à s'emboîter par complémentarité de formes.

9. Dispositif selon l'une des revendications 1 à 9, **caractérisé en ce que** le tuyau élastique (10) présente une ou plusieurs fentes (19) s'étendant, dans sa position détendue, en particulier axialement sur au moins une partie de sa longueur, laquelle ou lesquelles sont - déformées approximativement en une ou plusieurs hélices lors de la rotation des parties de manchon (7, 8).

10. Dispositif selon l'une des revendications 1 à 9, **caractérisé en ce que** le côté extérieur des parties de manchon (7, 8) est rendu rugueux ou pourvu de rayures, de stries ou de profilages.

11. Dispositif selon l'une des revendications 1 à 10, **caractérisé en ce qu'**une rainure annulaire (20) ou analogue est prévue sur le côté extérieur d'au moins une des parties de manchon (7, 8) pour l'application de matériel de suture pour fixer le dispositif (1) dans la poche de stimulateur.
